Europäisches Patentamt

European Patent Office

Office européen des brevets

(19)

(11) Publication number: **0 005 978**

A1

## EUROPEAN PATENT APPLICATION

(12)

(21) Application number: **79300958.0**

(22) Date of filing: 25.05.79

(51) Int. Cl.²: **G 01 N 33/16**
**A 61 B 10/00**

(30) Priority: 26.05.78 US 910060

(43) Date of publication of application:
12.12.79 Bulletin 79/25

(84) Designated Contracting States:
AT BE CH DE FR GB IT NL SE

(71) Applicant: WARNER-LAMBERT COMPANY
201 Tabor Road
Morris Plains, New Jersey 07950(US)

(72) Inventor: Babson, Arthur Lawrence
Old Mill Road
Chester, New Jersey 07930(US)

(74) Representative: Jones, Michael Raymond et al,
Haseltine Lake & Co. 28 Southampton Buildings
Chancery Lane
London WC2A 1AT(GB)

(54) Process for detecting and quantifying an antigen or antibody.

(57) A process is disclosed for detecting or quantifying an antigen or antibody in a sample, in which, in a dilute suspension under agitation, the rate of aggregation of antibody-coated particles or antigen-coated particles as measured by an increase in light transmission is a function of antigen or antibody concentration. Preferably, the particles which are coated are latex particles. The process can be applied as an immunoassay technique in which one or both of antigen and antibody concentrations may be determined by use of latex agglutination aggregometry. The process of the present invention is more sensitive than conventional slide agglutination techniques.

EP 0 005 978 A1

-1-

PROCESS FOR DETECTING AND QUANTIFYING AN ANTIGEN OR

ANTIBODY

This invention relates to a process for detecting and quantifying an antigen or antibody.

Previously, quantitative immunoassay techniques have fallen into a hierarchy of sensitivities in which precipitin reactions have shown the least sensitivity and radioimmunoassays have shown the greatest sensitivities.

Precipitin reactions involving antibody-containing gels, whether employing unidirectional diffusion in columns as in the Oudin Agar Column Procedure (Oudin, 1946, Compt. Rend. Acad. Sci. 222,115) or the Mancini radial immunodiffusion (G. Mancini, A.O.Carbonara and J.F.Heremans, "Immunochemical Quantitation of Antigens by Single Radial Immunodiffusion" in Immunochemistry 2:235-254 (1965)) have the further disadvantage of being slow and providing poor quantitation of the antigenic component. Quantitation and speed are somewhat improved with the Laurell technique (C.B.Laurell, "Quantitative Estimation of Proteins By Electrophoresis in Agarose Gel Containing Antibodies", Anal. Biochem. 15:45-52 (1966)), in which the reaction is carried out under the influence of an electric field, i.e. immunoelectrophoresis; however, the technique requires sophisticated equipment specifically designed for this procedure.

Precipitin reactions have also been quantified in solution after reaction of polyvalent antigens with a specific antibody by nephalometric measurement (L.M. Killingsworth and J. Savory, "Nephalometric Studies of the Precipitin Reaction: A Model System for Specific Protein Measurements", Clin. Chem. 19:403 (1973)) or turbidimetric measurement. A kinetic measurement of the precipitin reaction has also been described (A.J.Buffone, J.Savory, R.E.Cross, and J.E.Hammont, "Evaluation of Kinetic Light Scattering As An Approach to the Measurement of Specific Proteins With The Centrifugal Analyzer", I. Methodology Clin. Chem. 21:1731 (1975)) as a means to quantify immunoglobulins.

There are a number of other disadvantages associated with precipitin reactions. Besides lacking sensitivity, precipitin reactions in solution are only valid over a discrete concentration range, because either an antigen or antibody excess will cause what is known as the Ehrlich prozone phenomenon in which the precipitin reaction does not occur. Also, all solutions required for the reaction must be perfectly clear as any traces of turbidity or cloudiness may obscure the results.

Attaching antibodies to solid support systems such as latex particles or erythrocytes enhances the sensitivity of the reaction 100-1000 fold compared to the precipitin reaction and avoids or reduces the prozone phenomenon when there is an excess of antibody present.

Agglutination reactions of this type are basically qualitative procedures. Agglutination reactions using latex particles have only been quantified by a technique known as differential light scattering (P.Blume and L.J. Greenberg, "Application of Differential Light Scattering To The Latex Agglutination Assay For Rheumatoid Factor", Clin. Chem. 21:1234 (1975)). This so-called Blume technique, however, requires a polarizing laser light source and very sophisticated detection devices to measure the intensity of scattered light at the various angles of scatter. The equipment and technology required to quantify agglutination reactions in this manner therefore make the procedures not economically feasible for the small to medium clinical laboratory.

According to the present invention, there is provided

## 0005978

-3-

a process for detecting and quantifying an antigen or antibody in a sample, which process comprises mixing the sample containing a quantity of an antigen or antibody with a suspension kept under agitation of reactive antibody-coated particles or antigen-coated particles, and allowing or causing the suspension of resulting antigen-antibody-coated particle reaction product or antibody-antigen-coated particle reaction product to agglutinate, thereby indicating the presence of a specific antigen or antibody in the sample; measuring the change in percentage of light transmission through the reaction product suspension for a predetermined period of time; and comparing the thus measured change in percentage transmission with a standard graph indicating the percentage transmission for the same predetermined time period for varying quantities of antigen or antibody.

One aspect of the present invention is based on the discovery that, in dilute suspension and under constant agitation, the rate of aggregation of antibody-coated particles as measured by an increase in light transmission is a function of antigen concentration. Moreover, the present invention provides a quantitative determination of antigen concentration with about a 10-fold increase in sensitivity over the conventional slide agglutination technique.

Thus, with the present invention the rate of change of light transmission in a dilute suspension of antibody-coated particles under constant agitation is a function of antigen concentration. The present invention is therefore applicable to the quantitation of polyvalent antigens.

Another aspect of the present invention is based on the discovery that the rate of change of light transmission in a dilute suspension of antigen-coated particles under constant agitation is a function of antibody concentration. The present invention is therefore applicable to the quantitation of antibodies.

Examples of polyvalent antigens which are easily quantified at low concentrations using the process of the present invention are various coagulation factors such as fibrinogen, fibrinogen degradation products, factor VIII antigen, antithrombin III, plasminogen, and platelet factor 4; various serum proteins such as immunoglobulin G, immunoglobulin A, immunoglobulin M, $\alpha$-antitrypsin, $\beta$-lipoprotein, $\alpha_2$-macroglobulin, thryoxine-binding globulin, C'3 complement, transferrin, ceruloplasmin, haptoglobulin, and human placental lactogen; and various urinary proteins such as human chorionic gonadotropin and Bence Jones protein.

Examples of antibodies which are easily quantified at low concentrations using the process of the present invention with antigen-coated particles are antistreptolyxin O, rheumatoid factor and antinuclear antibody.

As the particles used to bind and carry the antigen or antibody molecules, there can be used any compatible material such as glass, clay, red blood cells or polystyrene latex particles, these materials being known as such. Of these materials, polystyrene latex particles having a particle size in the range from 0.15 to 0.9 micron are preferred for their ease of use.

The present invention can be used in immunological quantitative and qualitative assays for various antigen and antibody systems.

The following Examples are merely illustrative of the present invention and are not intended to limit the invention. In the Examples reference is made to the accompanying drawings in which:-

Figure 1 represents graphically the relationship between the milligrams of Fibrinogen per 100 ml of plasma, and the change in value of the percentage transmission ($\Delta\%T$) over 4 minutes, for the experiment of Example 1;

Figure 2 represents graphically the relationship between the amount ($\mu$g) of fibrinogen degradation products (FDP) per millilitre of serum, and the reciprocal of the time in minutes to reach a change of 20 in the % transmission ($\Delta\%T=20$), for the experiment of Example 2; and

Figure 3 represents graphically the relationship

0005978

100 ml in the undiluted plasma.

## EXAMPLE 2

### FDP Assay

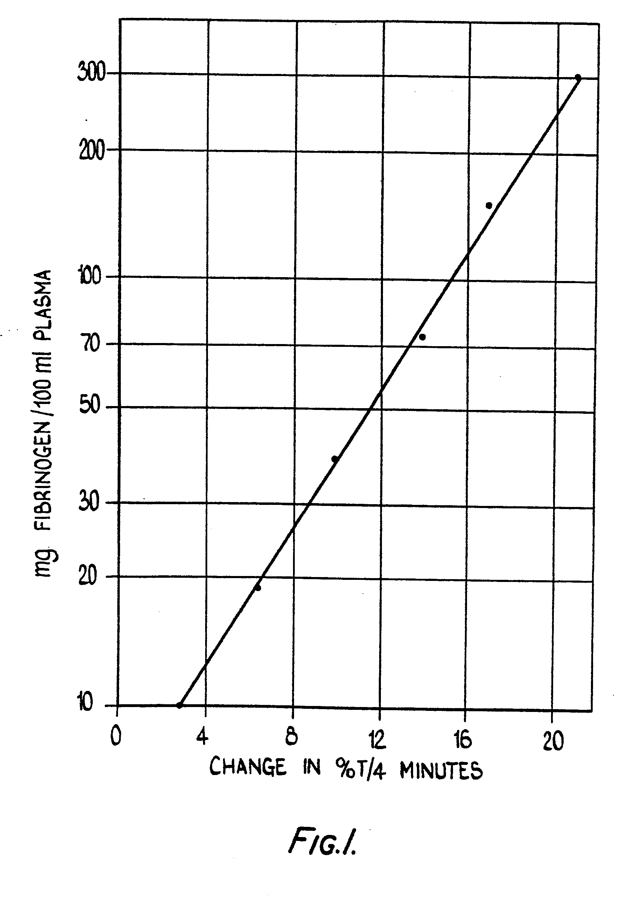

The excellent sensitivity of the assay for fibrinogen in plasma (as shown in Figure 1) suggested its use as a quantitative assay for fibrinogen degradation products (hereinafter abbreviated to FDP) in serum.

Normal plasma was serially diluted in serum. FDP-sensitized Latex prepared in a similar manner to that disclosed in United States Patent Specification No. 4,003,988, was diluted 1:50 in the same piperazine buffer as used in Example 1, and the reaction was initiated by the mixing of 1 ml of Suspension with 25 $\mu$l of serum.

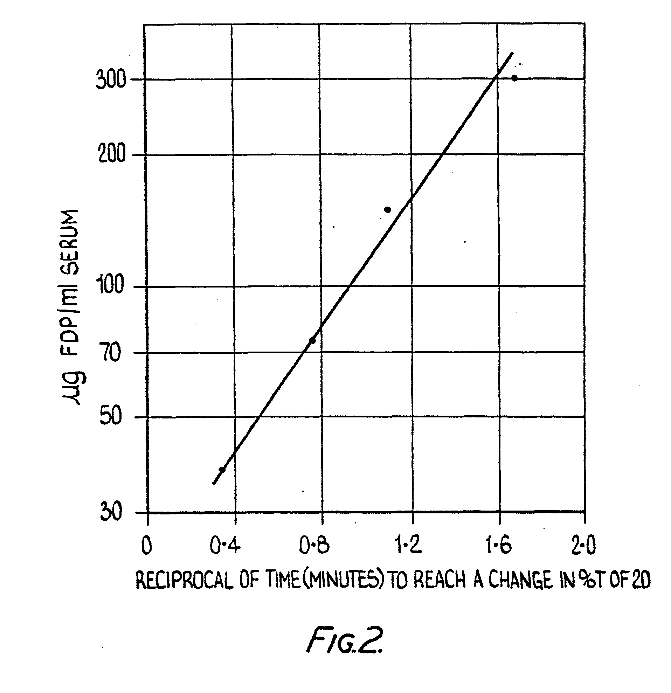

Figure 2 shows the dose response between 30 and 300 $\mu$g of FDP per ml of serum. In the assay, the reciprocal of the time in minutes to reach a change in 20 of the %T ($\Delta$%T=20) was used as the standard of measurement.

## EXAMPLE 3

### HCG Assay

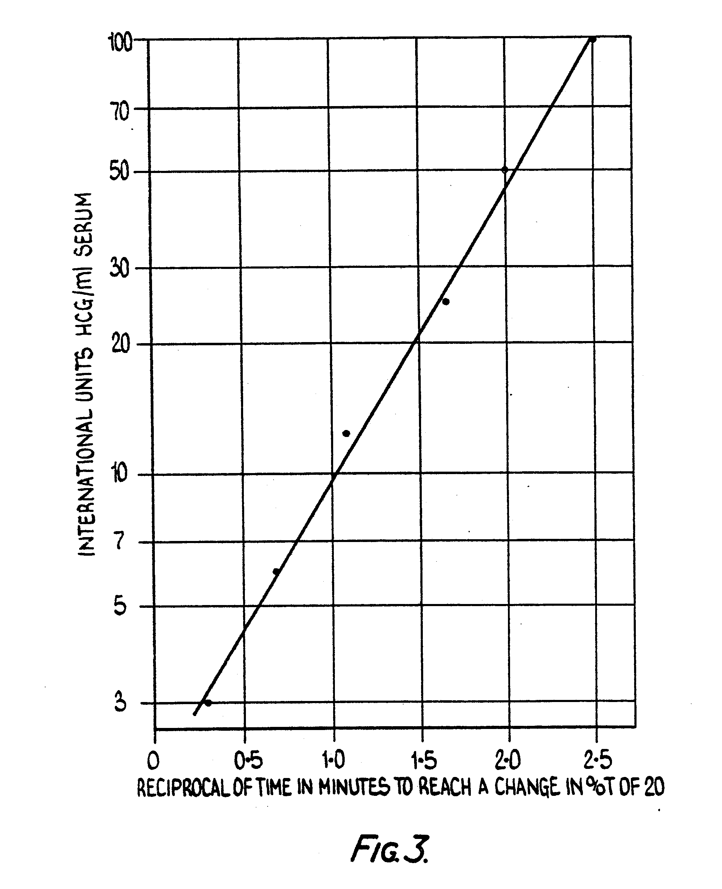

A human chorionic gonadotropin (HCG) assay in serum using the same conditions as in Example 2 gave a dose response curve between 3 and 100 units per ml as illustrated in Figure 3.

When using latex particles as the particles coated with antibody or antigen, the process, which can be regarded as kinetic latex agglutinometry, enables an unknown quantity of an antigen or antibody to be easily determined by assaying and comparing the results obtained against such standard curves as those shown in the figures of the accompanying drawings. Such a technique has several advantages over other immunoassay procedures, including latex agglutination on a slide, for example; it is a homogeneous procedure requiring only a single stable reagent.

between the amount (in international units) of human chorionic gonadotropin (HCG) per millilitre of serum, and the reciprocal of the time in minutes to reach a change of 20 in the % transmission (Δ%T=20), for the experiment of Example 3.

## EXAMPLE 1

Fibrinogen Assay

Rabbit Anti-Human Fibrinogen was prepared and adsorbed on latex particles in accordance with the procedure described in United States Patent Specification No. 4,003,988, the only difference being that the rabbits used to produce the antibody were presensitized to fibrinogen and not to HCG as in the patent disclosure.

The assay was performed on a Chronolog Platelet Aggregometer using the following parameters:

(1)  The recorder used in conjunction with the aggregometer was adjusted to a 5mv span (less than 5mv was found to give extraneous noise;  greater than 5mv reduced sensitivity) and a chart speed of 0.5 inch per minute.

(2)  The latex was diluted by a factor of 80 in a piperazine buffer (prepared as in United States Patent Specification No. 4,003,988) and the recorder was set to read 0%T at this dilution.  A dilution of 1:160 of latex in buffer was used to calibrate the recorder to register 100%T.  Although other dilutions are not excluded, these specific dilutions were chosen as giving a satisfactory degree of sensitivity in the particular instrument used in this series of tests.

(3)  Constant minimum stirring of the text reaction mixture was maintained in the aggregometer cuvette with a polytetrafluoroethylene (such as that sold under the registered Trade Mark "Teflon") stir bar.

The test was performed by placing 1ml of the diluted latex in an aggregometer cuvette and warming the cuvette to 27°C.  The antigen-antibody reaction was initiated by the addition of 50 μl of plasma diluted 1:100 in saline.

Figure 1 shows the relationship of fibrinogen concentration in plasma to the change in percentage transmission (Δ%T) over the course of four minutes.  A good dose response is evident between 100 and 300 mg fibrinogen per

CLAIMS:

1.    A process for detecting and quantifying an antigen
or antibody in a sample, which process comprises mixing
the sample containing a quantity of an antigen or antibody
with a suspension kept under agitation of reactive antibody-
coated particles or antigen-coated particles, and allowing
or causing the suspension of resulting antigen-antibody-
coated particle reaction product or antibody-antigen-
coated particle reaction product to agglutinate, thereby
indicating the presence of a specific antigen or antibody
in the sample;  measuring the change in percentage of light
transmission through the reaction product suspension for a
predetermined period of time;  and comparing the thus
measured change in percentage transmission with a standard
graph indicating the percentage transmission for the same
predetermined time period for varying quantities of antigen
or antibody.

2.    A process according to Claim 1, which includes the
use of antibody-coated particles of latex.

3.    A process according to Claim 1, which includes the
use of antigen-coated particles of latex.

4.    A process according to Claim 1, 2 or 3, wherein the
agitation is substantially constant.

Fig. I.

0005978

FIG.2.

FIG.3.

0005978

## European Patent Office — EUROPEAN SEARCH REPORT

Application number

EP 79 300 958.0

| | DOCUMENTS CONSIDERED TO BE RELEVANT | | CLASSIFICATION OF THE APPLICATION (Int. Cl.²) |
|---|---|---|---|
| Category | Citation of document with indication, where appropriate, of relevant passages | Relevant to claim | |
| X | DE - A - 2 736 805 (MITSUBISHI CHEMICAL INDUSTR.) <br> * claims 1, 6, 11 to 13; page 27, line 29 to page 29, line 14 * <br> & FR - A - 2 362 398 <br> — | 1-4 | G 01 N 33/16 <br> A 61 B 10/00 |
| | DE - A - 2 749 956 (F. HOFFMANN-LA ROCHE) <br> * claims 1, 3, 6, 19 to 21, examples 1 to 4 * <br> & FR - A - 2 370 972 <br> — | 1-3 | **TECHNICAL FIELDS SEARCHED (Int.Cl.²)** <br><br> A 61 B 10/00 <br> G 01 N 33/16 |
| | DE - A - 2 204 684 (F. HOFFMANN-LA ROCHE) <br> * claims 1, 6, pages 2 to 6 * <br> & FR - A - 2 125 000 <br> — | 1-3 | |
| A | US - A - 4 080 264 (MASSACHUSETTS INSTITUTE OF TECHNOLOGY) <br> * whole document * <br> ——— | | **CATEGORY OF CITED DOCUMENTS** <br><br> X: particularly relevant <br> A: technological background <br> O: non-written disclosure <br> P: intermediate document <br> T: theory or principle underlying the invention <br> E: conflicting application <br> D: document cited in the application <br> L: citation for other reasons <br><br> &: member of the same patent family, corresponding document |

The present search report has been drawn up for all claims

| Place of search | Date of completion of the search | Examiner |
|---|---|---|
| Berlin | 02-08-1979 | SCHWARTZ |

EPO Form 1503.1   06.78